# NEUE EUROPÄISCHE PATENTSCHRIFT

(11) **EP 0 360 848 B2**
(45) Veröffentlichungstag und Bekanntmachung des Hinweises auf die Entscheidung über den Einspruch: **12.07.2000**
(45) Hinweis auf die Patenterteilung: 04.08.1993
(21) Anmeldenummer: 89902645.4
(22) Anmeldetag: 23.02.1989
(51) Int. Cl.: C25D 3/48, C25D 3/62, A61K 6/04

(54) **Verwendung eines Goldbades zur Herstellung von Zahnersatz**
Use of a gold bath for the preparation of dental prosthesis
Utilisation d'un bain d'or pour la fabrication de prothèses dentaires

(30) Priorität: 24.02.1988 DE 3805627
(43) Veröffentlichungstag der Anmeldung: 04.04.1990
(73) Patentinhaber: Wieland Edelmetalle GmbH & Co., 75179 Pforzheim (DE)
(72) Erfinder: SCHNEIDER, Thomas, D-7537 Remchingen (DE); PFISTERER, Anita, D-7531 Kieselbronn (DE); BOLCH, Thomas, D-7101 Albstadt (DE); GEMMLER, Armin, D-7910 Neu-Ulm (DE)
(74) Vertreter: Patentanwälte Ruff, Beier, Schöndorf und Mütschele
(86) Internationale Anmeldenummer: EP8900164
(87) Internationale Veröffentlichungsnummer: WO8908156

(56) Entgegenhaltungen:
- DE-C- 2 723 910
- DE-C- 2 829 980
- US-A- 3 787 463
- G.A. SOMERS UND J.BIAU "CLINICAL APPLICATIONS OF ELECTROFORMED GOLD ALLOYS", SUR/FIN '87, INTERNATIONAL CONFERENDE PROCEEDINGS, 13.-16.7.87, SESSION A, SEITEN 1-14
- J.Setz u.a. "Galvano-keramische Kronen, Herstellung und erste Erfahrungen", dental-labor XXXVI, Heft 1/88, Seiten 71-74.

## Beschreibung

Die Erfindung betrifft die Verwendung eines wäßrigen Gold- oder Goldlegierungsbads. Das Bad enthält dabei Gold in Form eines Goldsulfit-Komplexes, ggf. mindestens ein Legierungsmetall, mindestens eine lösliche Nitrosäure mit mindestens einer Nitrogruppe, bei der der Säurerest ein Carbonsäurerest und/oder ein Sulfonsäurerest ist, welche in Form der Säure, eines Derivates oder eines Salzes vorliegen, sowie sonst übliche Badzusätze.

Die Möglichkeit des Einsatzes von Goldsulfit-Komplexen zur galvanischen Goldabscheidung ist schon lange bekannt. Allerdings erlangten diese Bäder aufgrund ihrer mangelnden Stabilität lange Zeit keine praktische Bedeutung. Da aber wegen der Toxizität der üblicherweise verwendeten Bäder auf Goldcyanid-Basis ein starkes Interesse an deren Ersatz bestand, wurden auf dem Gebiet der Goldbäder auf Sulfitbasis kontinuierlich weitere Anstrengungen unternommen.

Die US-Patentschrift 3 057 789 beschreibt ein Bad zur Goldabscheidung, das einen Goldsulfit-Komplex und als weiteren Komplexbildner zusätzlich Ethylendiamin-tetraessigsäure enthält. Der Komplexbildner ist zur Stabilisierung nötig, um überhaupt Gold aus dem in dieser Patentschrift beanspruchten Bad abscheiden zu können. Als Sulfite werden zur Herstellung des Bades bevorzugt Natrium- und Kaliumsulfit eingesetzt. Die Stabilität dieses Bades bereitet, vor allem im schwach sauren pH-Bereich, große Schwierigkeiten und außerdem ist eine Abscheidung von Goldlegierungen nicht möglich. Dies hängt damit zusammen, daß das Bad nur im alkalischen pH-Bereich verwendbar ist.

Auch die deutsche Offenlegungsschrift 16 21 180 beschreibt ein Bad auf der Basis von Goldsulfit, das aber ebenfalls nur im alkalischen pH-Bereich betrieben werden kann. Deshalb können mit diesem Bad keine hochlegierten Goldniederschläge erhalten werden und außerdem auch nur eine begrenzte Anzahl von unedlen Metallen als Legierungskomponente mit abgeschieden werden.

Die deutsche Auslegeschrift 22 44 437 befaßt sich lediglich mit einem wäßrigen elektrolytischen Bad, das zur Abscheidung von Gold-Palladium-Legierungen vorgesehen ist. Es enthält ebenfalls ein Alkali-Goldsulfit sowie Palladium in einer Komplexverbindung. Auch diese Druckschrift beschränkt sich auf einen im alkalisch liegenden pH-Wert, was eine Anwendung dieses Bades auf andere Legierungselemente unmöglich macht.

Die deutsche Patentschrift 28 29 980 schlägt zur Verwendung für die Abscheidung von Gold und Goldlegierungen ein wäßriges Bad vor, das die Goldverbindung in Form eines Ammonium-Goldsulfit-Komplexes enthält. Zur weiteren Stabilisierung des Bades wird der Vorschlag gemacht, dem Bad bevorzugt Pikrinsäure zuzusetzen. Die in dieser Druckschrift beschriebenen Maßnahmen machen es zwar möglich, das Bad bei schwach sauren pH-Werten zu betreiben, aber die Handhabbarkeit des Bades ist nicht in gewünschter Weise einfach. Das liegt daran, daß die zugesetzte Pikrinsäure sich während des Abscheidevorgangs nicht proportional mit der geflossenen Ladung abbaut. Um die Pikrinsäure ersetzen zu können, müßte man deshalb eine aufwendige Analytik betreiben, wodurch die Badführung aber sehr kompliziert wird. Des weiteren entsteht in einem solchen Bad nach gewisser Zeit ein Überschuß an Dithionit, der zu einer unerwünschten, sich selbst katalysierenden Goldabscheidung im Bad führt. Dies hat zur Folge, daß das Bad nach der Patentschrift 28 29 980 keine gleichmäßigen Qualitäten der abgeschiedenen Schichten liefern kann. Diese Qualitäten sind aber u.a. für die Verwendung bei der Zahnersatzherstellung eine unabdingbare Voraussetzung.

Aus der Veröffentlichung von G.A. Somers et al.: "Clinical Applications of Electroformed Gold Alloys", SUR/FIN '87, International Technical Conference Proceedings, 13. bis 16. Juli 1987, Chicago, Illinois, Session A, Seiten 1 bis 14 sind ebenfalls Bäder bekannt, die Gold in Form eines Goldsulfit-Komplexes und ggf. mindestens ein Legierungsmetall enthalten. In dieser Druckschrift ist die Verwendung wäßriger Gold- oder Goldlegierungsbäder zur Herstellung von Zahnersatz in Verbindung mit zu brennender Keramik zur Verblendung angesprochen.

In der FR-A-2353656 ist ein Zusatzgemisch für Bäder zum elektrolytischen Abscheiden von Gold oder Goldlegierungen beschrieben. Dieses Gemisch enthält definierte organische wasserlösliche Nitroverbindungen sowie mindestens eine wasserlösliche Verbindung von Arsen, Antimon, Wismut, Thallium und/oder Selen. Bei den Nitroverbindungen kann es sich um Nitrosäuren handeln, bei denen der Säurerest ein Carbonsäurerest oder ein Sulfonsäurerest ist. Das Zusatzgemisch kann zusätzlich weitere Bestandteile enthalten, die üblicherweise in Goldbädern zur Anwendung gelangen. Bei den Bädern kann es sich um Goldcyanidbäder oder um Bäder auf Basis von Goldsulfit handeln. Das Gemisch wird Bädern zugesetzt, die in der Elektronikindustrie verwendet werden.

Zahl und Größe der kritischen Parameter sind bei der elektrolytischen Abscheidung von Gold- und Goldlegierungs-Schichten nämlich stark vom anschließenden Verwendungszweck abhängig. So sind zum Beispiel die Konzentrationsbereiche für verschiedene Badzusätze und die Parameter für die Badführung bei der Verwendung der Schichten für dekorative Zwecke wesentlich weniger kritisch, als wenn man die Schichten beispielsweise zur Herstellung von Zahnersatz in Verbindung mit Keramik benutzen will. Hierbei muß die abgeschiedene Metallschicht nämlich brennstabil sein, um die Keramik auf diese aufbrennen zu können. Deshalb besteht ein Bedürfnis nach Bädern für die galvanoplastische Herstellung von Zahnersatz, da hierfür brennstabile Schichten konstanter Qualität benötigt werden. Dies ist der Grund dafür, daß der Zahnersatz bisher hauptsächlich durch gußtechnische Verfahren in Verbindung mit Keramik hergestellt wird.

Die Erfindung hat also die Aufgabe, ein wäßriges Bad zur Abscheidung von Gold oder Goldlegierungen bei der Herstellung von Zahnersatz in Verbindung mit zu brennender Keramik verwendbar zu machen, das hochwertige Schichten gleichbleibender Qualität liefert. Bei dem Bad soll eine Variation des pH-Wertes über einen großen Bereich möglich sein, um den Zusatz verschiedenster Legierungsmetalle zu ermöglichen und hochlegierte Goldlegierungen herzustellen. Neben der konstant hohen Qualität der abgeschiedenen Gold- oder Goldlegierungs-Schichten soll das Bad zusätzlich eine möglichst einfache Handhabung, hohe Stromausbeuten und leichte Ergänzbarkeit der Reagentien ermöglichen. Außerdem soll das Bad eine hohe Lebensdauer besitzen.

Es war überraschend, daß durch den Einsatz eines wäßrigen Bads der obengenannten Art eine Verwendung der damit abgeschiedenen Metallschichten als Zahnersatz in Verbindung mit aufzubrennender Keramik zur Verblendung möglich wird.

Für die Herstellung von mit Keramik verblendetem Zahnersatz benötigt man, wie schon beschrieben, brennstabile Metallschichten gleichmäßig guter Qualität. Diese Anforderung wird durch die erfindungsgemäße Verwendung erfüllt. Die Keramik läßt sich durch nachträgliches Brennen auf die Metallschicht aufbringen. Damit kann auf die gußtechnische Herstellung von Zahnersatz in Verbindung mit Keramik verzichtet werden und die Vorteile des galvanoplastischen Verfahrens gegenüber dem Gußverfahren, wie gute Reproduktion von Details und komplexer Formen, Möglichkeit der Herstellung desselben Objekts in mehreren Exemplaren, Reduktion des Gewichts und damit des Preises u.a., kommen voll zum Tragen. Bei der Anwendung des galvanoplastischen Verfahrens zur Herstellung von Zahnersatz unter Verwendung des Bades wird eine höhere Paßgenauigkeit als bei der bisher dominierenden Metall-Gußtechnik erreicht. Die erhaltenen Schichten, die wesentlich dicker sein müssen als die in der Elektronikindustrie benötigten, sind in Verbindung mit Dentalkeramik brennstabil. Eine ausgezeichnete Anpassung des galvanoplastisch hergestellten Zahnersatzes (z.B. Krone oder Brücke) an die üblichen Dental-Keramikmassen ist möglich. Dies gilt insbesondere im Hinblick auf die thermische Ausdehnung und den dadurch möglichen dauerhaften Verbund, die geringe Wärmespannung und die verminderte Rißneigung der Keramik. Ein galvanisch geformter Zahnersatz ermöglicht einen besseren Ausgleich der möglichen Ungleichheiten eines Befestigungswerkstoffes (Zement, Kleber) für den Zahnersatz, als dies bei mit dem Gußverfahren hergestelltem Zahnersatz der Fall ist. Dadurch werden mechanische Verformungen beim Eingliedern des Zahnersatzes besser aufgefangen.

Das im folgenden beschriebene Bad ist besonders zur Herstellung von Zahnersatz nach dem Verfahren geeignet, wie es in der Veröffentlichung "Galvano-keramische Kronen, Herstellung und erste Erfahrungen" von J. Setz u.a. in dental-labor, XXXVI, Heft 1/88, 71-74 beschrieben ist. Es wird auch auf das dort aufgeführte Literaturverzeichnis verwiesen.

Die dem wäßrigen Bad zugesetzte lösliche Nitrosäure besitzt einen Säurerest, der ein Carbonsäurerest und/oder ein Sulfonsäurerest ist. Dieser Säurerest kann direkt in Form der Säure, in Form eines Derivates der Säure oder in Salzform vorliegen. Bevorzugt handelt es sich bei den Derivaten des Säurerests um die Amid-, Imid- oder Amidinform. Beispiele für solche Nitrosäuren bzw. -Derivate sind die 2- oder 3-Nitrobenzoesäure sowie deren Amide, die 3,5-Dinitrobenzoesäure und m-Nitrobenzamidin-hydrochlorid.

Die bei der Erfindung eingesetzten Nitrosäuren enthalten mindestens eine, insbesondere aber 1 bis 3 Nitrogruppen. Bevorzugt sind solche Nitrosäuren, die mindestens einen aromatischen Kern besitzen, wobei wiederum die Nitrosäuren besonders vorteilhaft eingesetzt werden können, die nur einen aromatischen Kern aufweisen. Vorzugsweise wird als aromatische Nitrosäure eine solche verwendet werden, bei der sowohl mindestens eine Nitrogruppe als auch mindestens eine Sulfonsäuregruppe an einen aromatischen Kern, insbesondere an denselben aromatischen Kern, gebunden sind. Dabei kann eine solche aromatische Nitrosäure insbesondere eine Nitrobenzolsulfonsäure mit 1 bis 3 Nitrogruppen, vorzugsweise eine solche mit 1 oder 3 Nitrogruppen, sowie einer Sulfonsäuregruppe sein. Außerdem kann diese Nitrobenzolsulfonsäure ggf. noch weitere Substituenten am Benzolkern besitzen.

Die Zugabe der Nitrosäure in das Bad kann sowohl zusammen mit dem Goldsulfit-Komplex als auch getrennt erfolgen. Außerdem kann die Menge an Nitrosäure, die dem wäßrigen Bad zugegeben wird, direkt auf den Goldgehalt des Bades bezogen werden.

Das nach der Erfindung verwendete Bad enthält das Gold in der Form eines Goldsulfit-Komplexes, wobei die Goldkonzentration (bezogen auf metallisches Feingold) in der Regel zwischen 1 und 50 g/l Bad, vorzugsweise zwischen 2 und 20 g/l Bad, liegt.

Der pH-Wert des Bades kann beispielsweise in Abhängigkeit von der Zugabe an Legierungsmetallen und anderen Badzusätzen auf Werte zwischen 4 und 12, vorzugsweise zwischen 6 und 12, insbesondere zwischen 6 und 8, eingestellt werden. Dies kann mit Phosphatpuffer, Boratpuffer, Citratpuffer und anderen in der Galvanotechnik verwendbaren Puffersystemen geschehen. In dem genannten pH-Bereich ist das Bad einsetzbar, die Einsatztemperatur liegt gewöhnlich zwischen 20 °C und 80 °C, vorzugsweise zwischen 40 °C und 60 °C.

Als Legierungsmetalle kommen alle die bekannten Metalle des Periodensystems in Frage, die mit Gold zusammen aus einer wäßrigen Lösung abscheidbar sind. Insbesondere sind dies jedoch die Edelmetalle und von diesen vorzugsweise Palladium und Ruthenium.

Das erfindungsgemäß verwendbare wäßrige Bad zur Abscheidung von Gold oder Goldlegierungen kann, insbesondere zusätzlich zu den Nitrosäuren, mindestens einen wasserlöslichen organischen Komplexbildner enthalten. Dieser wasserlösliche organische Komplexbildner kann vorzugsweise für die Komplexbildung der ggf. vorhandenen Legierungsmetalle, insbesondere der Edelmetalle, zugegeben sein.

Bei dem zugesetzten Komplexbildner handelt es sich vorzugsweise um mindestens eine wasserlösliche organische Stickstoffverbindung und dabei insbesondere um mindestens einen Stickstoff-Heterocyclus. Als Stickstoff-Heterocyclus können nach der Erfindung bevorzugt aromatische Stickstoff-Heterocyclen eingesetzt werden. Besonders vorteilhaft ist dabei die Verwendung von Pyridin.

Dem Bad können außerdem in bekannter Weise die Elemente Kupfer und Antimon als Glanzbildner für die Abscheidung dickerer Schichten zugegeben werden. Antimon kann dabei zum Beispiel in Form seines Tartrats eingesetzt werden, während Kupfer als Kupfersulfat unter Zusatz von EDTA (Ethylendiamintetraessigsäure bzw. deren Natriumsalz) Verwendung finden kann.

Wenn das Bad frei von Palladium und/oder anderen Legierungsmetallen ist, enthält es besonders bevorzugt Antimon, insbesondere im Bereich von 50 bis 150 mg/l. Antimontartrat ist bevorzugt. Es wurde gefunden, daß die Abscheidung von Gold in Gegenwart von Antimon zu höherglänzenden Niederschlägen führt. Dies ist vermutlich darauf zurückzuführen, daß Antimon in hydrolysierter Form in der Lösung suspendiert vorliegt. Der Hydrolysegrad kann durch Zugabe von Komplexbildner für Antimon bzw. von Hydrophobika beeinflußt werden. Ein besonders bevorzugtes Hydrophobikum ist Butylglykolsulfat.

Ein auf diese Weise zusammengesetztes Bad weist gegenüber anderen Bädern auf Goldsulfit-Basis Vorteile auf. So sind bei diesem Bad nicht nur die Konzentrationsspielräume für die Badzusätze Kupfer und Antimon wesentlich größer, sondern es lassen sich auch höhere Stromdichten von bis zu 2 A/dm² bei der Abscheidung verwenden.

Eine weitere wichtige Verbesserung zeigt sich darin, daß die Menge an Nitrosäure proportional mit dem Strom und damit mit der abgeschiedenen Goldmenge abgebaut wird. Dies führt dazu, daß sich sowohl die Nitrosäure als auch der Goldsulfit-Komplex in einfacher Weise ergänzen lassen, was die Badführung vereinfacht. Eine Eigenschaft des Bades ist auch, daß keine Selbstabscheidung des Goldes mehr erfolgt. Im Gegensatz zu anderen Goldsulfitbädern wie z.B. einem solchen mit Pikrinsäurezusatz findet sich im Bad analytisch kein Dithionit mehr. Diese Dithionitfreiheit macht das Bad, wie oben beschrieben, ergänzbar und führt somit zu einer wesentlichen Erhöhung der Lebensdauer und Ausbeutbarkeit des Bades.

Aber nicht nur bei der Verwendbarkeit der abgeschiedenen Schichten aus dem beschriebenen Bad zur Herstellung von galvanoplastisch geformtem Zahnersatz, sondern auch bei der einfachen Badführung zeigen sich die Vorteile der Erfindung.

### Beispiele

Die in den folgenden Beispielen aufgeführten Bestandteile wurden in den angegebenen Mengen gelöst und die Lösung mit Wasser auf 1 l aufgefüllt. Bei den im einzelnen Beispiel angegebenen Badparametern, wie Temperatur und pH-Wert wurde eine Zahnprothese vergoldet bzw. mit einer Goldlegierung beschichtet. Die Zahnprothese und der Elektrolyt wurden während der Abscheidung in Bewegung gehalten und der Elektrolyt kontinuierlich filtriert. Als Anode wurde das im Beispiel angegebene Material verwendet.

### Zusammensetzung 1 :

40 ml/l Ammoniumsulfitlösung 34 %
40 g/l Ammoniumsulfat (NH₄)₂SO₄
20 g/l Ammoniumhydrogenphosphat (NH₄)₂HPO₄
10 g Gold als Komplex
100 mg/l 2,4,6-Trinitrobenzolsulfonsäure
40 mg/l Kupfercarbonat
50 mg/l Ethylendiamintetraessigsäure
100 mg/l Antimontartrat
Temperatur: 55 - 65 °C
pH-Wert: 7,5 - 8
Strom: ca. 0,5 A/dm²
Anoden: Platiniertes Titan

### Zusammensetzung 2:

20 ml/l Ammoniumsulfitlösung 34 %
20 g/l Ammoniumsulfat (NH₄)₂SO₄
20 g/l Ammoniumhydrogenphosphat (NH₄)₂HPO₄
15 g Gold als Komplex
100 mg/l 3-Nitrobenzolsulfonsäure-Natriumsalz
50 mg/l Kupfercarbonat
80 mg/l Ethylendiamintetraessigsäure
120 mg/l Antimontartrat
Temperatur: 60 - 65 °C
pH-Wert: 7,5
Strom: 0,4 A/dm²
Anoden: Rutheniumoxidanoden

### Zusammensetzung 3:

10 ml/l Ammoniumsulfitlösung
80 g/l Ammoniumsulfat (NH₄)₂SO₄
60 g/l Nitrilotriessigsäure-Trinatriumsalz
15 g Gold als Komplex
300 mg/l 2,4,6-Trinitrobenzolsulfonsäure
80 mg/l Kupfercarbonat
160 mg/l N-Hydroxyethyl-Ethylendiamintriessigsäure
Temperatur: 50 - 60 °C
pH-Wert: 8 - 9
Strom: 0,4 - 0,6 A/dm²
Anoden: Platiniertes Titan

### Zusammensetzung 4:

10 g/l Ammoniumhydrogenphosphat (NH₄)₂HPO₄
100 g/l Ammoniumsulfat (NH₄)₂SO₄
15 g/l Kaliumnatriumtartrat
10 g Gold als Komplex
100 mg/l Trinitrobenzolsulfonsäure
100 mg/l 3-Nitrobenzolsulfonsäure-Natriumsalz
100 mg/l Kupfercarbonat
200 mg/l Diethylentriaminpentaessigsäure
Temperatur: 55 °C
pH-Wert: 9 - 10
Strom. 0,2 A/dm²
Anoden: Platiniertes Titan

Sämtliche Bäder konnten während des Betriebes ohne Verminderung der Abscheidungsqualität stark erschöpft werden. Durch Zugabe des Goldsulfit-Komplexes sowie, bezogen auf die Goldmenge, der Nitrosäure und ggf. weiterer verbrauchter Badbestandteile konnte das Bad regeneriert werden.

Die erhaltenen Goldniederschläge waren glatt und glänzend sowie ausnahmslos geeignet, unter mehrmaligem Auftragen keramischer Schichten gebrannt zu werden.

In ähnlicher Weise wurde mit den folgenden Badzusammensetzungen gearbeitet:

### Zusammensetzung 5:

200 g/l Ammoniumsulfat (NH₄)₂SO₄
50 g/l Ammoniumhydrogenphosphat (NH₄)₂HPO₄
10 g/l Kaliumnatriumtartrat
8 g/l Gold als Komplex
200 mg/l 4-Nitrobenzylpyridin
150 mg/l Kupfercarbonat
300 mg/l Diethylentriaminpentaessigsäure
350 mg/l Antimontartrat
Temperatur: 50 °C
pH-Wert: 7,8 - 8,5
Strom: Ca. 0,2 A/dm²
Anoden: Rutheniumoxidanoden

### Zusammensetzung 6:

100 ml/l Ammoniumsulfitlösung 34 %
40 g/l Nitrilotriessigsäure-Trinatriumsalz
20 g/l Ammoniumsulfat (NH₄)₂SO₄
8 g/l Gold als Komplex
100 mg/l 2,4,5-Trinitrobenzolsulfonsäure
2 g/l Palladium als Komplex
0,5 - 2 g/l Pyridinderivat, insbesondere Pyridin
5 g/l Glycin
125 mg/l Kupfercarbonat
Temperatur: 55 - 58 °C
pH-Wert: 7,8
Strom: 0,3 - 0,6 A/dm²
Anoden: Platinierte Titananoden

Auch für diese Zusammensetzungen des Bades zeigten sich die erfindungsgemäßen Verbesserungen in Bezug auf Badführung und Brennstabilität.

## Patentansprüche

1. Verwendung eines wäßrigen Gold- oder Goldlegierungsbads, enthaltend
- Gold in Form eines Goldsulfit-Komplexes,
- ggf. mindestens ein Legierungsmetall,
- mindestens eine lösliche Nitrosäure mit mindestens einer Nitrogruppe, bei der der Säurerest ein Carbonsäurerest und/oder ein Sulfonsäurerest ist, welche in Form der Säure, eines Derivates oder eines Salzes vorliegen, sowie
- sonst übliche Badzusätze,
zur Herstellung von Zahnersatz in Verbindung mit zu brennender Keramik zur Verblendung.

2. Verwendung nach Anspruch 1, dadurch gekennzeichnet, daß es sich bei dem Derivat um ein Amid-, Imid- oder Amidinderivat handelt.

3. Verwendung nach Anspruch 1 oder 2, dadurch gekennzeichnet, daß die Nitrosäure 1 bis 3 Nitrogruppen besitzt.

4. Verwendung nach einen der vorhergehenden Ansprüche, dadurch gekennzeichnet, daß die Nitrosäure mindestens einen aromatischen Kern besitzt, wobei ein aromatischer Kern bevorzugt ist.

5. Verwendung nach einem der vorhergehenden Ansprüche, dadurch gekennzeichnet, daß die Nitrosäure sowohl mindestens eine an einen aromatischen Kern gebundene Nitrogruppe als auch mindestens eine an einen aromatischen Kern gebundene Sulfonsäuregruppe enthält.

6. Verwendung nach einem der vorhergehenden Ansprüche, dadurch gekennzeichnet, daß die Nitrosäure eine Nitrobenzolsulfonsäure mit 1 bis 3 Nitrogruppen ist, wobei 1 und 3 Nitrogruppen bevorzugt sind, und die Nitrobenzolsulfonsäure ggf. noch weitere Substituenten am Benzolkern besitzt.

7. Verwendung nach einem der vorhergehenden Ansprüche, dadurch gekennzeichnet, daß die Menge an Nitrosäure auf den Goldgehalt des Bades bezogen ist.

8. Verwendung nach einem der vorhergehenden Ansprüche, dadurch gekennzeichnet, daß der pH-Wert des Bades zwischen 4 und 12, vorzugsweise zwischen 6 und 12, insbesondere zwischen 6 und 8 liegt.

9. Verwendung nach einem der vorhergehenden Ansprüche, dadurch gekennzeichnet, daß das Bad mindestens einen wasserlöslichen organischen Komplexbildner enthält.

10. Verwendung nach einem der vorhergehenden Ansprüche, dadurch gekennzeichnet, daß das Bad mindestens eine wasserlösliche organische Stickstoffverbindung, insbesondere mindestens einen Stickstoff-Heterocyclus, enthält.

11. Verwendung nach Anspruch 10, dadurch gekennzeichnet, daß der Stickstoff-Heterocyclus ein aromatischer Heterocyclus, insbesondere Pyridin, ist.

12. Verwendung nach einem der vorhergehenden Ansprüche, dadurch gekennzeichnet, daß das Bad zusätzlich mindestens ein Hydrophobikum, insbesondere Butylglykolsulfat, enthält.

13. Zahnersatz, erhältlich bei der Verwendung des wäßrigen Gold- oder Goldlegierungsbades nach einem der vorhergehenden Ansprüche.

## Claims

1. Use of an aqueous gold or gold alloy bath containing gold in the form of a gold sulphite complex, optionally at least one alloying metal, at least one soluble nitro acid with at least one nitro group, in which the acid residue is a carboxylic and/or sulphonic acid residue, which is present in the form of the acid, a derivative or a salt and otherwise conventional bath additives, for producing dental prostheses, in conjunction with ceramic to be fired for veneering purposes.

2. Use according to claim 1, characterized in that the derivative is an amide, imide or amidine derivative.

3. Use according to claim 1 or 2, characterized in that the nitro acid has 1 to 3 nitro groups.

4. Use according to one of the preceding claims, characterized in that the nitro acid has at least one aromatic nucleus, one aromatic nucleus being preferred.

5. Use according to one of the preceding claims, characterized in that the nitro acid contains at least one nitro group bound to an aromatic nucleus and at least one sulphonic acid group bound to an aromatic nucleus.

6. Use according to one of the preceding claims, characterized in that the nitro acid is a nitrobenzene sulphonic acid with 1 to 3 nitro groups, 1 and 3 nitro groups being preferred, and the nitrobenzene sulphonic acid optionally has further substituents on the benzene nucleus.

7. Use according to one of the preceding claims, characterized in that the nitro acid quantity is related to the bath gold content.

8. Use according to one of the preceding claims, characterized in that the pH-value of the bath is between 4 and 12, preferably between 6 and 12 and particularly between 6 and 8.

9. Use according to one of the preceding claims, characterized in that the bath contains at least one water-soluble, organic sequestrant.

10. Use according to one of the preceding claims, characterized in that the bath contains at least one water-soluble, organic nitrogen compound, particularly at least one nitrogen heterocycle.

11. Use according to claim 10, characterized in that the nitrogen heterocycle is an aromatic heterocycle, particularly pyridine.

12. Use according to one of the preceding claims, characterized in that the bath also contains at least one hydrophobic agent, particularly butyl glycol sulphate.

13. Dental prosthesis obtainable by using the aqueous gold or gold alloy bath according to one of the preceding claims.

## Revendications

1. Utilisation d'un bain aqueux d'or ou d'alliage d'or comprenant:
- de l'or, sous forme d'un complexe de sulfite d'or;
- le cas échéant, au moins un métal d'alliage;
- au moins un acide nitrique soluble, comportant au moins un groupe fonctionnel nitrique, dont le résidu d'acide consiste en un résidu d'acide carboxylique et/ou un résidu d'acide sulfonique, lesquels se présentent sous forme d'acide, de dérivé ou de sel, ainsi que
- tous les ingrédients de bain habituels,
en vue de la production de prothèses dentaires, en conjonction avec de la céramique à cuire au four, servant de revêtement.

2. Utilisation conformément à la revendication 1, caractérisée par le fait que le dérivé consiste en un dérivé amidé, imidé ou amidiné.

3. Utilisation conformément à l'une des revendications 1 ou 2, caractérisée par le fait que l'acide nitrique possède 1 à 3 groupes fonctionnels nitriques.

4. Utilisation conformément à l'une des revendications ci-dessus, caractérisée par le fait que l'acide nitrique possède au moins un noyau aromatique, possédant de préférence un seul noyau aromatique.

5. Utilisation conformément à l'une des revendications ci-dessus, caractérisée par le fait que l'acide nitrique comporte aussi bien au moins un groupe fonctionnel nitrique lié à un noyau aromatique, qu'au moins un groupe fonctionnel sulfonique lié à un noyau aromatique.

6. Utilisation conformément à l'une des revendications ci-dessus, caractérisée par le fait que l'acide nitrique consiste en un acide sulfonique de nitrobenzène comportant 1 à 3 groupes fonctionnels nitriques, de préférence 1 ou 3 groupes fonctionnels, et que l'acide sulfonique de nitrobenzène possède, le cas échéant, d'autres substituants au niveau de son noyau benzénique.

7. Utilisation conformément à l'une des revendications ci-dessus, caractérisée par le fait que la quantité d'acide nitrique est fonction de la teneur en or du bain.

8. Utilisation conformément à l'une des revendications ci-dessus, caractérisée par le fait que le pH du bain se situe entre 4 et 12, de préférence entre 6 et 12, et notamment entre 6 et 8.

9. Utilisation conformément à l'une des revendications ci-dessus, caractérisée par le fait que le bain contient au moins un agent complexant organique hydrosoluble.

10. Utilisation conformément à l'une des revendications ci-dessus, caractérisée par le fait que le bain contient au moins un composé azoté hydrosoluble organique, et notamment au moins un hétérocycle azoté.

11. Utilisation conformément à l'une des revendications ci-dessus, caractérisée par le fait que le hétérocycle azoté consiste en un hétérocycle aromatique, notamment de pyridine.

12. Utilisation conformément à l'une des revendications ci-dessus, caractérisée par le fait que le bain contient de plus au moins un agent hydrophobe, notamment du sulfate de glycol butylique.

13. Prothèse dentaire, obtenue par l'utilisation du bain aqueux d'or ou d'alliage d'or conformément à l'une des revendications ci-dessus.
